# EUROPEAN PATENT SPECIFICATION

(11) **EP 1 011 700 B1**
(45) Date of publication and mention of the grant of the patent: **04.09.2002**
(21) Application number: 98924072.6
(22) Date of filing: 08.06.1998
(51) Int. Cl.: A61K 35/74, A61K 7/28, A61P 1/02

(54) **AN ORAL CARE COMPOSITION COMPRISING A BACILLUS PULLULANASE AND A DEXTRANASE**
ZUSAMMENSETZUNG ZUR MUNDHYGIENE, DIE EINE BAKTERIELLE PULLULANASE UND DEXTRANASE ENTHÄLT
COMPOSITION D'HYGIENE BUCCO-DENTAIRE RENFERMANT UNE BACILLUS PULLULANASE ET UNE DEXTRANASE

(30) Priority: 17.06.1997 DK 71097
(43) Date of publication of application: 28.06.2000
(73) Proprietor: Novozymes A/S, 2880 Bagsvaerd (DK)
(72) Inventor: TSUCHIYA, Rie, DK-2880 Bagsvaerd (DK); NIELSEN, Peder, Holk, DK-2880 Bagsvaerd (DK); AASLYNG, Dorrit, DK-2880 Bagsvaerd (DK)
(86) International application number: DK9800238
(87) International publication number: WO98057653

(56) References cited:
- DE-A- 1 965 043

## Description

### FIELD OF THE INVENTION

The present invention relates to a composition comprising a *Bacillus* pullulanase in combination with a dextranase.

The invention also relates to the use of a pullulanase of Ba*cillus* origin in combination with a dextranase in oral care compositions and products and further the *Bacillus* pullulanase for use as a medicament for the removal of dental plaque and/or preventing the formation of dental plaque.

### BACKGROUND OF THE INVENTION

The formation of dental plaque leads to dental caries, gingival inflammation, periodontal disease, and eventually tooth loss. Dental plaque is a mixture of bacteria, epithelial cells, leukocytes, macrophages, and other oral exudate. Said bacteria produce highly branched polysaccharides which together with microorganisms from the oral cavity form an adhesive matrix for the continued proliferation of plaque.

As plaque continues to accumulate rock-hard white or yellowish deposits arise. These deposits are called calcified plaque, calculus or tartar, and are formed in the saliva from plaque and minerals, such as in particular calcium.

### Oral polysaccharides

Oral polysaccharides are produced from sucrose introduced into the mouth, *e.g.* as a food or beverage constituent, by the action of cariogenic microorganisms, such as *Streptococcus mutans* or *Streptococcus sanguis,* growing in the oral cavity.

Said oral polysaccharides comprise water-soluble dextran, having large portions of α-1,6 glucosidic linkage, and a major component of water-insoluble extra-cellular polysaccharides called "mutan" comprised of a backbone with α-1,3-glycosidic linkages and branches with α-1,6-glycosidic linkages.

Mutan binds to hydroxyapatite (constituting the hard outer porous layer of the teeth) and to acceptor proteins on the cell surface of said cariogenic bacteria adhering to the teeth surface.

### Pullulanase

Pullulanases are α-Dextrin endo-1,6-α-glucosidases (or pullulan 6-glucano-hydrolase) which hydrolysis 1,6-α-D-glucosidic linkages in pullulan, amylopectin and glycogen. Several microorganisms are known to be capable of producing pullulanase, including bacteria of the genus *Aerobacter* (Bender et al., (1959), Biochim Biophys. Acta 36, p. 309), the genus *Bacillus* (EP 063,909 from Novo Nordisk A/S and EP 605,040 from Solvay SA), the genus *Fervidobacterium (WO 92*/*16617* from Novo Nordisk A/S), the genus *Pyrococcus* (WO 92/02614 from Novo Nordisk A/S).

Commercially available pullulanases products include Promozyme® from Novo Nordisk, which is a heat stable debranching enzyme obtained from a strain *of Bacillus acidopullulyticus* used for the saccharification step in dextrose and maltose production. Another commercial pullulanase is Optimax® (Solvay SA) obtainable from *Bacillus deramificans.*

### Dextranase

Dextranases are α-1,6-glucanases (also known as 1,6-α-D-glucan 6 glucanohydrolases) which degrade the α-1,6-glycosidic linkages in dextran. Several microorganisms are capable of producing dextranases, among them fungi of *Penicillium, Paecilomyces, Aspergillus, Fusarium, Spicaria, Verticillium, Helminthosporium* and the *Chaetomium* genera; bacteria of the genera *Lactobacillus, Streptococcus, Cellvibrio, Cytophaga, Brevibacterium, Pseudomonas, Corynebacterium, Arthrobacter* and *Flavobacterium* and yeasts such as *Lipomyces starkeyi.*

Commercially available products include Dextranase 50 L from Novo Nordisk A/S produced by fermentation of strains of *Penicillium lilacium*. Dextranase 50 L is used in the sugar industry to break down dextran in raw sugar juice or syrup.

To prevent the formation of dental caries, plaque, and tartar, it has been suggested to add a pullulanase and/or a dextranase and/or other enzymes to oral care compositions and products.

DE 1,965,043 (Aspro-Nicholas Ltd.) describes an oral care composition comprising a dextranase and a pullulanase.

### SUMMARY OF THE INVENTION

It is the object of the present invention to provide improved oral care products which safely (*i.e.* without harming the tissue and structure of the oral cavity) and effectively prevent the formation of dental plaque and/or remove already deposited dental plaque.

The present inventors have found that compositions and products comprising a pullulanase derived from a strain of the bacterial genus *Bacillus* in combination with a dextranase give a synergistic effect on mutan hydrolysis and on dental plaque removal and prevention of dental plaque formation.

In the first aspect the invention relates to a composition comprising a *Bacillus* pullulanase and a dextranase.

In the second aspect the invention relates to an oral care product comprising an oral care composition of the invention.

It is also the object of the invention to provide the use of *Bacillus* pullulanases for in vitro hydrolysing mutan containing material.

In a final aspect the invention relates to a *Bacillus* pullulanase for use as a medicament and the use of a *Bacillus* pullulanase for the manufacture of a medicament for preventing the formation of dental plaque and/or removing dental plaque already formed.

### BRIEF DESCRIPTION OF THE DRAWING

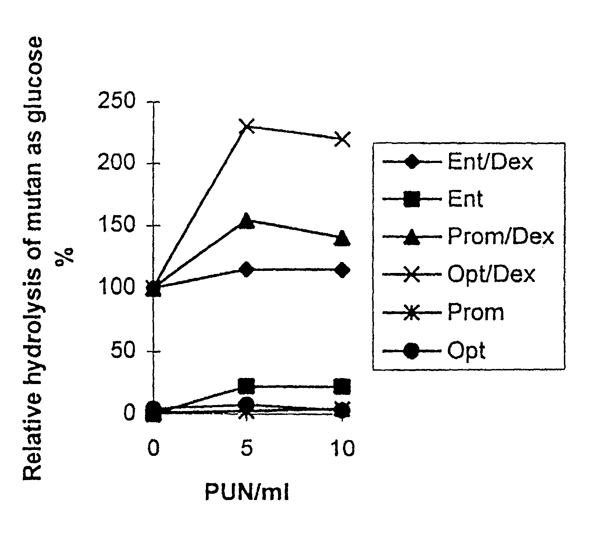

Figure 1 compares the mutan-hydrolysing effect of two *Bacillus* pullulanases and a *Enterobacter aerogenes* pullulanase alone and in combination with *Paecilomyces lilacinum* dextranase.

### DETAILED DESCRIPTION OF THE INVENTION

It is the object of the present invention to provide improved oral care products.
"Improved" oral care products mean products which are safe (*i.e.* do not harm the tissue and structure of the oral cavity) and which more effectively prevent the formation of dental plaque and/or remove already deposited dental plaque.

As mentioned above oral care compositions comprising a pullulanase and a dextranase are known.

The present inventors have surprisingly found that pullulanases derived from the bacteria genus *Bacillus* in combination with a dextranase may be used for providing improved oral care products. Such oral care products remove dental plaque more effectively than other corresponding products comprising other bacterial pullulanases in combination with a dextranase.

A synergistic effect between a *Bacillus* pullulanase and a dextranase is obtained in connection with dental plaque removal. When combining a standard bacterial pullulanase, such as the En*terobacter aerogenes* pullulanase and a dextranase and/or a mutanase the pullulanase does not contribute to the plaque removing effect.

Therefore, compositions and products comprising the combination of a *Bacillus* pullulanase and a dextranase have a number of advantages in comparison to corresponding compositions.

The amount of enzymes needed to obtain the desired effect may be reduced and/or less time is need to obtain the desired effect.

The reduced amount of enzymes needed is of commercial interest for oral care product manufacturers, as the costs of producing such product, according to the invention, can be reduced.

Further, if the original amount of enzymes is added to the product, an improved product is obtained as the desired effect will often be obtained within a shorter period of time.

Also the user of an oral care product of the invention (which will be described in more details below) prepared from an oral care composition of the invention, will benefit from the present invention, as the direct and indirect disadvantages (*e.g.* yellow deposits on the teeth and prevention of dental holes and gingivitis, respectively) can be prevented safely and more effectively than with corresponding prior art products comprising a pullulanase and a dextranase.

It is to be understood that said oral care products may also directly or indirectly have other oral care functions at the same time, *e.g.* prevention of dental holes or gingivitis.

### Compositions

In the first aspect the invention relates to a composition comprising a *Bacillus* pullulanase (*i.e.* a pullulanase derived from a bacteria of the genus *Bacillus*) and a dextranase. An oral care composition of the invention further comprises ingredients used in oral care compositions. Such ingredients are well know by the skilled person.

Specifically contemplated *Bacillus* pullulanases include pullulanases derived from a strain of *Bacillus acidopullulyticus* or a strain of *Bacillus deramificans.* However, also other *Bacillus* pullulanases may be used.

*Bacillus* pullulanases may advantageously be used in combination with a dextranase derived from *Paecilomyces sp.,* in particular *Paecilomyces lilacinus, or Pencillium sp.* such as *Penicillium lilacinus* or *Penicillium minioluteum* or another of the dextranases referred to in the "Background of the invention" section.

In a preferred embodiment of the invention the enzymes (*i.e.* especially the pullulanase and the dextranase) are recombinant enzymes.

The enzymes of the invention should be substantially active at the temperatures and pHs prevailing in the mouth of humans or animals *i.e.* between 20°C and 40°C, especially around 37°C and at pHs in the range from 5-9, especially from pH 6-8.5, or should be substantially active at temperatures and pHs prevailing in the mouth when using the specific oral care products in question.

In the context of the invention "substantially active" means that the relative enzymatic activity of the enzyme(s) is(are) above 50% in particular above 60%, especially above 70%, such as 90%, at the pH prevailing in the mouth when using the oral care composition in comparison to the relative activity at the pH optimum.

The oral care composition of the invention may further comprise one or more enzymes selected from the group of mutanases, oxidases, such as glucose oxidase, L-amino acid oxidase, peroxidases, such as *e.g.* the *Coprinus sp.* peroxidases described in WO 95/10602 (from Novo Nordisk A/S) or lactoperoxidaseor, haloperoxidases, laccases, proteases, such as papain, acidic protease (*e.g.* the acidic proteases described in WO 95/02044 (Novo Nordisk A/S)), endoglucosidases, lipases, amylases, including amyloglucosidases, such as AMG (from Novo Nordisk A/S), anti-microbial enzymes, and mixtures thereof.

An oral care composition of the invention may suitably have incorporated an amount of *Bacillus* pullulanase in an amount equivalent to an enzyme activity, calculated as enzyme activity units in the final oral care product, in the range from 0.001 PUN to 1000 PUN/ml, preferably from 0.01 PUN/ml to 500 PUN/ml, especially from 0.1 PUN/ml to 100 PUN/ml.

A dextranase may optionally, according to the invention, be incorporated in the oral care composition in an amount equivalent to an enzyme activity, calculated as enzyme activity units in the final oral care product, in the range from 0.001 KDU to 1000 KDU/ml, preferably from 0.01 KDU/ml to 500 KDU/ml, especially from 0.1 KDU/ml to 100 KDU/ml.

An oral care composition of the invention may further suitably comprise an amount of mutanase, such as *Tricoderma harzianum* mutanase, equivalent to an enzyme activity, calculated as enzyme activity units in the final oral care product, in the range from 0.001 MU to 1000 MU/ml, preferably from 0.01 MU/ml to 500 MU/ml, such as from 0.1 MU/ml to 100 MU/ml, especially 0.05 MU/ml to 100 MU/ml.

### Oral care products

The invention also relates to oral care products comprising an oral care composition of the invention. The oral care product may have any suitable physical form (*i.e.* powder, paste, gel, liquid, ointment, tablet etc.).

An "oral care product" can be defined as a product which can be used for maintaining or improving the oral hygiene in the mouth of humans and animals, by preventing formation of dental plaque, removing dental plaque, preventing and/or treating dental diseases etc.

At least in the context of the present invention oral care products do also encompass products for cleaning dentures, artificial teeth and the like.

Examples of such oral care products include toothpaste, dental cream, gel or tooth powder, odontic, mouth washes, pre- or post brushing rinse formulations, chewing gum, lozenges, and candy.

Toothpastes and tooth gels typically include abrasive polishing materials, foaming agents, flavouring agents, humectants, binders, thickeners, sweetening agents, whitening/bleaching/stain removing agents, water, and optionally enzymes.

Mouth washes, including plaque removing liquids, typically comprise a water/alcohol solution, flavour, humectant, sweetener, foaming agent, colorant, and optionally enzymes.

### Abrasives

Abrasive polishing material might also be incorporated into the dentifrice product of the invention. According to the invention said abrasive polishing material includes alumina and hydrates thereof, such as alpha alumina trihydrate, magnesium trisilicate, magnesium carbonate, kaolin, aluminosilicates, such as calcined aluminum silicate and aluminum silicate, calcium carbonate, zirconium silicate, and also powdered plastics, such as polyvinyl chloride, polyamides, polymethyl methacrylate, polystyrene, phenol-formaldehyde resins, melamine-formaldehyde resins, urea-formaldehyde resins, epoxy resins, powdered polyethylene, silica xerogels, hydrogels and aerogels and the like. Calcium pyrophosphate, water-insoluble alkali metaphosphates, dicalcium phosphate and/or its dihydrate, dicalcium orthophosphate, tricalcium phosphate, particulate hydroxyapatite and the like are also suitable as abrasive agents. It is also possible to employ mixtures of these substances.

Dependent on the oral care product the abrasive product may be present from 0 to 70% by weight, preferably from 1% to 70%. For toothpastes the abrasive material content typically lies in the range from 10% to 70% by weight of the final toothpaste product.

Humectants are employed to prevent loss of water from *e.g.* toothpastes. Suitable humectants for use in oral care products according to the invention include the following compounds and mixtures thereof: glycerol, polyol, sorbitol, polyethylene glycols (PEG), propylene glycol, 1,3-propanediol, 1,4-butanediol, hydrogenated partially hydrolysed polysaccharides and the like. Humectants are in general present from 0% to 80%, preferably 5 to 70% by weight in toothpaste.

Silica, starch, tragacanth gum, xanthan gum, extracts of Irish moss, alginates, pectin, cellulose derivatives, such as hydroxyethyl cellulose, sodium carboxymethyl cellulose and hydroxypropyl cellulose, polyacrylic acid and its salts, polyvinylpyrrolidone, can be mentioned as examples of suitable thickeners and binders, which helps stabilizing the dentifrice product. Thickeners may be present in toothpaste creams and gels in an amount from 0.1 to 20% by weight, and binders to the extent from 0.01 to 10% by weight of the final product.

### Foaming agents

As foaming agent soap, anionic, cationic, non-ionic, amphoteric and/or zwitterionic surfactants can be used. These may be present at levels of from 0% to 15%, preferably from 0.1 to 13%, more preferably from 0.25 to 10% by weight of the final product.

### Surfactants

Surfactants are only suitable to the extent that they do not exert an inactivation effect on the present protease. Surfactants include fatty alcohol sulphates, salts of sulphonated mono-glycerides or fatty acids having 10 to 20 carbon atoms, fatty acid-albumen condensation products, salts of fatty acids amides and taurines and/or salts of fatty acid esters of isethionic acid.

### Sweetening agents

Suitable sweeteners include saccharin.

### Flavouring agents

Flavours, such as spearmint, are usually present in low amounts, such as from 0.01% to about 5% by weight, especially from 0.1% to 5%.

### Whitening/bleaching agents

Whitening/bleaching agents include H₂O₂ and may be added in amounts less that 5%, preferably from 0.25 to 4%, calculated on the basis of the weight of the final product.

The whitening/bleaching agents may be an enzyme, such as an oxidoreductase. Examples of suitable teeth bleaching enzymes are described in WO 97/06775 (Novo Nordisk).

### Water

Water is usually added in an amount giving *e.g.* toothpaste a flowable form.

### Additional agents

Further water-soluble anti-bacterial agents, such as chlorhexidine digluconate, hexetidine, alexidine, quaternary ammonium anti-bacterial compounds and water-soluble sources of certain metal ions such as zinc, copper, silver and stannous (*e.g.* zinc, copper and stannous chloride, and silver nitrate) may also be included.

Also contemplated according to the invention is the addition of compounds which can be used as fluoride source, dyes/colorants, preservatives, vitamins, pH-adjusting agents, anti-caries agents, desensitizing agents etc.

### Enzymes

Other essential components used in oral care products and in oral care products of the invention are enzymes. Enzymes are biological catalysts of chemical reactions in living systems. Enzymes combine with the substrates on which they act forming an intermediate enzyme-substrate complex. This complex is then converted to a reaction product and a liberated enzyme which continues its specific enzymatic function.

Enzymes provide several benefits when used for cleansing of the oral cavity. Proteases break down salivary proteins, which are adsorbed onto the tooth surface and form the pellicle, the first layer of resulting plaque. Proteases along with lipases destroy bacteria by lysing proteins and lipids which form the structural components of bacterial cell walls and membranes. Dextranase breaks down the organic skeletal structure produced by bacteria that forms a matrix for bacterial adhesion. Proteases and amylases, not only prevents plaque formation, but also prevents the development of calculus by breaking-up the carbohydrate-protein complex that binds calcium, preventing mineralization.

### Toothpaste

A toothpaste produced from an oral care composition of the invention (in weight % of the final toothpaste composition) may typically comprise the following ingredients:

| | |
|---|---|
| Abrasive material | 10 to 70% |
| Humectant | 0 to 80% |
| Thickener | 0.1 to 20% |
| Binder | 0.01 to 10% |
| Sweetener | 0.1% to 5% |
| Foaming agent | 0 to 15% |
| Whitener | 0 to 5% |
| Enzymes | 0.0001% to 20% |

In a specific embodiment of the invention the oral care product is a toothpaste comprising

| | |
|---|---|
| a) 10% to 70% | Abrasive material |
| b) 0 to 80% | Humectant |
| c) 0.1 to 20% | Thickener |
| d) 0.01 to 10% | Binder |
| e) 0.1% to 5% | Sweetener |
| f) 0 to 15% | Foaming agent |
| g) 0 to 5% | Whitener |
| i) 0.0001% to 20% | Enzymes. |

Said enzymes referred to under i) include pullulanases, dextranase and other enzymes described above known to be used in toothpaste and the like.

### Mouth Wash

A mouth wash produced from an oral care composition of the invention (in weight % of the final mouth wash composition) may typically comprise the following ingredients:

| | |
|---|---|
| 0-20% | Humectant |
| 0-2% | Surfactant |
| 0-5% | Enzymes |
| 0-20% | Ethanol |
| 0-2% | Other ingredients (*e.g.* flavour, sweetener |
| | active ingredients such as fluorides). |
| 0-70% | Water |

The mouth wash composition may be buffered with an appropriate buffer *e.g.* sodium citrate or phosphate in the pH-range 6-7.5.

The mouth wash may be in none-diluted form *(i.e.* must be diluted before use).

### Method of Manufacture

The oral care composition and products of the present invention can be made using methods which are common in the oral product area.

### Use

According to the present invention *Bacillus* pullulanases are used for hydrolyzing mutan and further for use as a medicament for dental plaque prevention and/or removal. The *Bacillus* pullulanases are preferably used in combination with a dextranase, sometimes also with a mutanase.

It is within the scope of the invention to use the *Bacillus* pullulanase in food, feed and/or pet food products, preferably in combination with a dextranase, and sometimes further with a mutanase.

### Method of Manufacture

The oral care composition and products of the present invention can be made using methods which are common in the oral product area.

### MATERIALS AND METHODS

### Materials

### Pullulanases:

Promozyme®: Pullulanase (available from Novo Nordisk) derived from *Bacillus acidopullulyticus* (described in EP 63,909).

Optimax® : Pullulanase (as described in EP 605,040 (Solvay SA) derived from *Bacillus deramificans.*

*Enterobacter aerogenes* pullulanase purchased from Amano, JP)

### Other enzymes:

Dextranase from *Paecilomyces lilacinum* (available from Novo Nordisk).

Mutanase from *Trichoderma harzianum* CBS 243.71(available from Novo Nordisk)

### Microorganisms:

*Streptococcus mutans* strain *CBS 350.71* (or OMZ 176)
*Actinomyces viscosus DSM 43329*
*Fusobacterium nucleatum subsp. polymorphum DSM 20482*

### Solutions

Britton-Robinson Buffer
Erythrosin B (Sigma)

### Equipment

Shaker (Eppndorf Thermomixer, Type 5436)
Chromameter CR-200 (Minolta).

### Preparation of hydroxyapatite disks

Hydroxyapatite disks are prepared by compressing 250 mg of hydroxyapatite in a disk die at about 5,900 kg (13,000 lbs) of pressure for 5 minutes. The disks are then sintered at 600°C for 4 hours and finally hydrated with sterile de-ionised water.

### Sterilisation of hydroxyapatite disks

HA disks are sterilised at 180°C for two hours, hydrated with the sterilised de-ionised water and placed in a lid of Nunc tube (10 ml volume).

### Preparation of Mutan

Mutan is prepared by growing *Streptococcus mutans* CBS 350.71 at pH 6.5, 37°C (kept constant), and with an aeration rate of 75 rpm in a medium comprised of the following components:

| | |
|---|---|
| NZ-Case | 6.5 g/liter |
| Yeast Extract | 6 g/liter |
| (NH₄)₂SO₄ | 20 g/liter |
| K₂PO₄ | 3 g/liter |
| Glucose | 50 g/liter |
| Pluronic PE6100 | 0.1 % |

After 35 hours, sucrose is added to a final concentration of 60 g/liter to induce glucosyltransferase. The total fermentation time is 75 hours. The supernatant from the fermentation is centrifuged and filtered (sterile). Sucrose is then added to the supernatant to a final concentration of 5 % (pH is adjusted to pH 7.0 with acetic acid) and the solution is stirred overnight at 37°C. The solution is filtered and the insoluble mutan is harvested on propex and washed extensively with deionized water containing 1% sodium benzoate, pH 5 (adjusted with acetic acid). Finally, the insoluble mutan is lyophilized and ground.

### Methods

Activity determination One Pullulanase Unit Novo (PUN) is defined as the amount of enzyme which hydrolyzes pullulan, liberating reducing carbohydrate with a reducing power equivalent to 1 micro-mol glucose per minute under the following standard conditions:

| | |
|---|---|
| Substrate | 0.2% pullulan |
| Temperature | 40°C |
| pH | 5.0 |
| Reaction time | 30 minutes |

A detailed description of the analysis method (AF 190) is available on request.

### Determination of dextranase activity (KDU)

One Kilo Novo Dextranase Unit (1 KDU) is the amount of enzyme which breaks down dextran forming reducing sugar equivalent to 1 g maltose per hour in Novo Nordisk's method for determination of dextranase based on the following standard conditions:

| | |
|---|---|
| Substrate | Dextran 500 (Pharmacia) |
| Reaction time | 20 minutes |
| Temperature | 40°C |
| pH | 5.4 |

A detailed description of Novo Nordisk's analytical method (AF 120) is available on request.

### Assessment of the plaque removing effect

The method used for assessing the plaque removal effect is based on the method described by Kao in JP2250816. According to the present method the hydroxyapatite disks are coated with a biofilm comprising three strain of oral micro-organisms *(Streptococcus mutans, Actinomyces viscosus* and *Fusobacterium nucleatum)*.

To test plaque removing effect 0.1 % Erythrosin B in PBS is used to stain plaque present on the hydroxyapatite disks red. The intensity of the red color (*i.e.* a*) is measured on a Chromameter CR-200. The max. a* value is 60. Values below that indicate a less intensive red color (*i.e.* less plaque present). If the a* value is determined to zero no red color is present (*i.e.* no plaque).

### EXAMPLES

### Example 1

### Mutan Hydrolysis

The hydrolysis of mutan by two *Bacillus* pullulanases and one standard bacteria pullulanase derived from *Enterobacter aero*genes were tested alone and in combination with *Paecilomyces lilacinum* dextranase.

A Mutan suspension prepared as described in the "Materials and Method" section was dispersed in deionized water with an ultrasonicater to form a 2.5% (w/v) substrate suspension.

The following enzyme solutions dissolved in a 10 mM BR buffer were prepared.
Optimax® (*Bacillus* pullulanase): 0, 5 and 10 PUN/ml
Promozyme® (*Bacillus* pullulanase): 0, 5 and 10 PUN/ml
*Enterobacter aerogenes* pullulanase: 0, 5 and 10 PUN/ml

The above enzyme solution was also combined with 8.5 DU/ml *Paecilomyces lilacinum* dextranase.

Further, 10 mM Britton-Robinson buffer solutions (pH 5.5) were also prepared.

250 µl of each of the above mentioned enzyme solutions and 250 µl buffer solution (pH 5.5) were mixed in a microcentrifuge tube. Immediately thereafter 750 µl of Mutan suspension was added, incubated at 37°C in a shaker at the maximum speed.

After exactly 15 minutes, 250 µl of a 0.5 N HCl was added to terminate the enzymatic reaction. A control was prepared by adding a 0.5 N HCl before addition of a substrate suspension. Each of the reaction mixtures were subjected to centrifugation.

The solubilized carbohydrates, in the obtained supernatant, was determined as glucose according to the anthrone reaction method (J.H. Roe, (1955), J. Biol. Chem. **212,** p. 335).

The result is displayed in Figure 1. From Figure 1 it can be seen that the two *Bacillus* pullulanases, Promozyme® and Optimax®, respectively, in combination with *Paecilomyces* dextranase exhibit synergistic effect when hydrolyzing mutan, while the standard *Enterobacter* pullulanase in combination with the same dextranase does not.

## Claims

1. A composition comprising a *Bacillus* pullulanase and a dextranase.

2. The composition according to claim 1 further comprising ingredients used in oral care compositions.

3. The composition of claims 1 or 2, wherein the pullulanase is derived from a strain of *Bacillus acidopullulyticus* or *Bacillus deramificans*.

4. The composition according to any of claims 1 to 3, wherein the dextranase is derived from a strain of *Paecilomyces sp.,* in particular *Paecilomyces lilacinum.*

5. The composition according to any of claims 1 to 4, wherein the enzymes are substantially active at temperatures between 20°C and 40°C, especially around 37°C.

6. The composition according to claim 5, wherein the relative enzymatic activity of the enzyme is above 50% in particular above 60%, especially above 70%, such as 90%, at the pH prevailing in the mouth when using the composition in comparison to the relative activity at the pH optimum.

7. The composition according to any of claims 1 to 6, further comprising one or more enzymes selected from the group of mutanases, oxidases, peroxidases, haloperoxidases, laccases, proteases, endoglucosidases, lipases, amylases, anti-microbial enzymes, and mixtures thereof.

8. An oral care product comprising a composition of any of claims 1 to 7.

9. The oral care product according to claim 8, being a dentifrice, such as a toothpaste, tooth powder or a mouth wash.

10. The oral care product according to claims 8 to 9 further comprising a dextranase, such as a dextranase derived from *Paecilomyces sp*., in particular *P. lilacinum.*

11. Use of a *Bacillus* pullulanase for in vitro hydrolysis of mutan containing material.

12. The use according to claim 11 wherein the *Bacillus* pullulanase is used in combination with a dextranase and/or a mutanase.

13. A *Bacillus* pullulanase for use as a medicament.

14. The *Bacillus* pullulanase according to claim 13 for preventing the formation of dental plaque and/or removing dental plaque already formed.

15. The *Bacillus* pullulanase according to claims 13 to 14, wherein the *Bacillus* pullulanase is used in combination with a dextranase and/or a mutanase.

16. Use of a *Bacillus* pullulanase for the manufacture of a medicament for preventing the formation of dental plaque and/or removing dental plaque already formed.

## Patentansprüche

1. Zusammensetzung enthaltend eine *Bacillus* Pullulanase und eine Dextranase.

2. Zusammensetzung nach Anspruch 1, weiter enthaltend Inhaltsstoffe, die in Mundpflegezusammensetzungen verwendet werden.

3. Zusammensetzung nach Ansprüchen 1 oder 2, wobei die Pullulanase von einem Stamm von *Bacillus acidopullulyticus* oder *Bacillus deramificans* stammt.

4. Zusammensetzung nach irgendeinem der Ansprüche 1 bis 3, wobei die Dextranase von einem Stamm von *Paecilomyces sp*., insbesondere *Paecilomyces lilacium* stammt.

5. Zusammensetzung nach irgendeinem der Ansprüche 1 bis 4, wobei die Enzyme im wesentlichen aktiv bei Temperaturen zwischen 20°C und 40°C sind, insbesondere bei um etwa 37°C.

6. Zusammensetzung nach Anspruch 5, wobei die relative enzymatische Aktivität des Enzyms oberhalb 50 %, insbesondere oberhalb 60 %, ganz besonders oberhalb 70 %, wie 90 %, bei dem pH ist, der im Mund vorherrscht, wenn die Zusammensetzung verwendet wird, im Vergleich zur relativen Aktivität am pH Optimum.

7. Zusammensetzung nach irgendeinem der Ansprüche 1 bis 6, weiter umfassend ein oder mehrere Enzyme ausgewählt aus der Gruppe aus Mutanasen, Oxidasen, Peroxidasen, Haloperoxidasen, Laccasen, Proteasen, Endoglucosidasen, Lipasen, Amylasen, Anti-mikrobielle Enzyme und Mischungen aus diesen.

8. Mundpflegeprodukt enthaltend eine Zusammensetzung nach irgendeinem der Ansprüche 1 bis 7.

9. Mundpflegeprodukt nach Anspruch 8, das ein Zahnpflegemittel ist, wie eine Zahnpasta, ein Zahnpuder oder ein Mundwasser.

10. Mundpflegeprodukt nach Ansprüchen 8 bis 9, weiter enthaltend eine Dextranase, wie eine Dextranase stammend von *Paecilomyces sp*., insbesondere *P. lilacinum.*

11. Verwendung einer *Bacillus* Pullulanase zur *in vitro* Hydrolyse von Mutan enthaltendem Material.

12. Verwendung nach Anspruch 11, wobei die *Bacillus* Pullulanase in Kombination mit einer Dextranase und/oder einer Mutanase verwendet wird.

13. Eine *Bacillus* Pullulanase zur Verwendung als Medikament.

14. *Bacillus* Pullulanase nach Anspruch 13 zur Verhinderung der Bildung von Zahnbelag und/oder zum Entfernen von bereits gebildetem Zahnbelag.

15. *Bacillus* Pullulanase nach Ansprüchen 13 bis 14, wobei die *Bacillus* Pullulanase in Kombination mit einer Dextranase und/oder einer Mutanase verwendet wird.

16. Verwendung einer *Bacillus* Pullulanase zur Herstellung eines Medikaments zur Verhinderung der Bildung von Zahnbelag und/oder zur Entfernung von bereits gebildetem Zahnbelag.

## Revendications

1. Une composition comprenant *Bacillus* pullulanase et une dextranase.

2. La composition selon la revendication 1 comprenant, en outre, des ingrédients utilisés dans les compositions de soins orales.

3. La composition selon la revendication 1 ou 2, dans laquelle la pullulanase est dérivée d'une souche de *Bacillus acidopullulyticus* ou de *Bacillus deramificans.*

4. La composition selon l'une quelconque des revendications 1 à 3, dans laquelle la dextranase est dérivée d'une souche de *Paecilomyces sp.,* en particulier de *Paecilomyces lilacinum.*

5. La composition selon l'une quelconque des revendications 1 à 4, dans laquelle les enzymes sont essentiellement actives aux températures comprises entre 20° C et 40° C, notamment autour de 37° C.

6. La composition selon la revendication 5, dans laquelle l'activité enzymatique relative de l'enzyme est supérieure à 50 %, en particulier supérieure à 60 % et, notamment, supérieure à 70 %, comme 90 %, au pH prévalant dans la bouche lorsqu'on utilise la composition comparativement à l'activité relative au pH optimum.

7. La composition selon l'une quelconque des revendications 1 à 6, comprenant, en outre, une ou plusieurs enzymes choisies parmi le groupe comprenant les mutanases, les oxydases, les peroxydases, les haloperoxydases, les laccases, les protéases, les endoglucosidases, les lipases, les amylases, les enzymes antimicrobiennes et leurs mélanges.

8. Un produit de soin oral comprenant une composition selon l'une quelconque des revendications 1 à 7.

9. Le produit de soin oral selon la revendication 8, qui est un dentifrice comme une pâte dentaire, une poudre dentaire ou un produit de lavage de la bouche.

10. Le produit de soin oral selon les revendications 8 à 9, comprenant, en outre, une dextranase, comme une dextranase dérivée de *Paecilomyces sp.,* en particulier *P. lilacinum.*

11. Utilisation d'une *Bacillus* pullulanase pour l'hydrolyse in vitro d'un matériau renfermant un mutan.

12. L'utilisation selon la revendication 11, dans laquelle la *Bacillus* pullulanase est utilisée en combinaison avec une dextranase et/ou une mutanase.

13. Une *Bacillus* pullulanase destinée à être utilisée comme un médicament.

14. La *Bacillus* pullulanase selon la revendication 13 pour empêcher la formation de plaque dentaire et/ou pour éliminer la plaque dentaire déjà formée.

15. La *Bacillus* pullulanase selon les revendications 13 à 14, dans laquelle la *Bacillus* pullulanase est utilisée en combinaison avec une dextranase et/ou une mutanase.

16. Utilisation d'une *Bacillus* pullulanase pour la fabrication d'un médicament pour empêcher la formation de plaque dentaire et/ou pour éliminer la plaque dentaire déjà formée.
